# EUROPEAN PATENT SPECIFICATION

(11) **EP 1 430 031 B1**
(45) Date of publication and mention of the grant of the patent: **03.12.2008**
(21) Application number: 02751512.1
(22) Date of filing: 20.05.2002
(51) Int. Cl.: C07D 223/12, C07D 223/16

(54) **KINETIC RESOLUTION OF A INTERMEDIATE USEFUL IN THE PRODUCTION OF BENAZEPRIL AND ANALOGUES THEREOF**
KINETISCHE AUFLÖSUNG EINES NÜTZLICHEN ZWISCHENPRODUKTS FÜR DIE HERSTELLUNG VON BENAZEPRIL UND SEINER ANALOGA
RESOLUTION CINETIQUE D'UN PRODUIT INTERMEDIAIRE UTILISE DANS LA PRODUCTION DE BENAZEPRIL ET D'ANALOGUES DE BENAZEPRIL

(30) Priority: 18.05.2001 US 291888 P; 19.07.2001 US 910509
(43) Date of publication of application: 23.06.2004
(73) Proprietor: ScinoPharm Taiwan, Ltd., Tainan Country 741, China R.O.C. (TW)
(72) Inventor: TSENG, Wei-Hong, Sang-Ming Area, Kao-Hsiung City (TW); SCHLOEMER, Georg, Sarasota, FL 34242 (US); CHENG, Kau, Ming, Tainan City (TW); CHEN, Chien, Wen, 744 Tainan County (TW); CHENG, Chih, Wen, Section 2, Tainan City (TW)
(74) Representative: Epping - Hermann - Fischer
(86) International application number: PCT/IB2002/003060
(87) International publication number: WO 2003/003972

(56) References cited:
- US-A- 4 410 520

## Description

### BACKGROUND OF THE INVENTION

### 1. Field of the Invention

The present invention relates to a process for the preparation of an angiotensin-converting enzyme (ACE) inhibitor useful in the treatment of hypertension, chronic heart failure and progressive chronic renal insufficiency. In particular, the processes of the present invention are useful for the commercial production of 3-[(1'-(alkoxycarbonyl)-3'-phenylpropyl)amino]-2-oxo-[1]-benzazepine and derivatives thereof. More notably, the present invention demonstrates an unique process of converting an intermediate compound in the synthesis of benazepril from one diastereomer form R,S to another diastereomer form S,S, which is the desired form for the production of benazepril by utilizing a process of epimerization and kinetic resolution of a class of chemical compounds.

### 2. Description of the Related Art

One of the most: popular ACE inhibitors is benazepril, 3-[(1-(ethoxycarbonyl)-3-phenyl-(1S)-propyl)amino]-2,3,4,5-tetrahydro-2-oxo-1H-10(3S)-benzazepine-1-acetic acid, which has a general formula as shown below and is generally marketed as the mono hydrochloride form (benazepril HCl) and administered orally in therapeutic use.

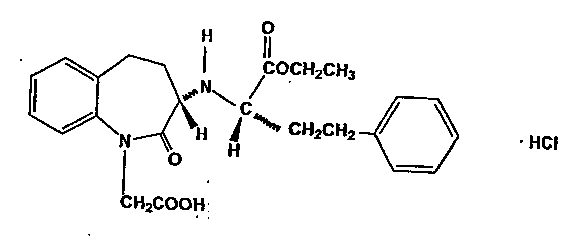

A number of previous publications and patents have disclosed processes for the preparation of benazepril, including Helvetica Chimica Acta (page 337, vol. 71, 1988), Journal of the Chemical Society: Perkin Transaction I (page 1011, 1986), U. S. Patent Nos. 4,410,520 (1983), 4,473,575 (1984), 4,575,503 (1986), 4,600,534 (1986), 4,785,089 (1988), 5,066,801 (1991), and 5,098,841 (1992)

Prior methods employ sulfonate esters of 2-(R)-hydroxy-4-phenyl butonate alkyl esters condensing with 3-(S)-amino-ε-caprolactam for the synthesis of benazepril. However, the use of sulfonate esters requires a chiral 2-(R)-hydroxy-4-phenyl butanate alkyl ester and an expensive leaving group in the form of a substituted phenyl sulfonate ester. Additionally, the reaction can sometimes lead to undesirable racemization.

U.S. Patent Nos 4,410,520 and 4,575,503 disclose processes for the preparation of 3-amino-[1]-benzazepin-2-one-1-alkanoic acids. The processes are complex by either first bonding a chiral amino group to the 1-position carbonyl group, or by attaching a chiral amino group to the 3-position carbon with a good leaving group through a two-step reaction. Consequently, the processes require several complex reaction steps, which may be more costly to conduct.

A simpler and more efficient process for preparing 3-[(1'-(alkoxycarbonyl)-3'-phenylpropyl)amino]-2-oxo-[1]-benzazepin-(1-alkyl acids) is desirable. The present invention discloses a process of directly bonding a chiral amino acid ester to the 3-position carbon of 2,3,4,5,-tetrahydro-1H-1-benzazepine-2-one derivatives to produce a mixture of diastereomers, and a novel epimerization process and resolution process to convert the undesired diastereomer form, R,S, into the desired diastereomer form S,S in high yield. The production of the S,S form of benazepril is thus greatly simplified.

For certain chemical compounds when used as therapeutic drugs, only one specific stereoisomer is effective. The other stereoisomers of the same compound may be less effective or have no effect at all. As a general technique, kinetic resolution (crystallization during epimerization) has been used to stereoselectively produce a single diastereomer from a mixture, see Marianne Langston, et. al, Organic Process Research & Development, 4:530-533 (2000). The success of this method depends on a large solubility difference between the diastereomers and a condition that facilitates effective epimerization of the desired optical center. Thus, in a predetermined solvent, the desired diastereomer should have low solubility and thus precipitate easily while the undesired diastereomer has a higher solubility and thus remains in the solution. As the desired diastereomer forms crystals, its concentration in the solution becomes lower, a condition that helps further conversion from the undesired diastereomer to the desired one.

The synthesis of benazepril, an important angiotensin converting enzyme (ACE) inhibitor, is made difficult due to the need to prepare the S,S diastereomeric form of the compound. The need to chemically resolve an early intermediate or to chemically synthesize a chiral intermediate adds much more expense to the synthesis. For example, Novartis Pharmaceuticals reported a process for the production of the (S)-chiral amine form based on a crystallization technique. While this method produced good yields of the desired s-chiral amine form, the necessary step of subsequently producing the (S)-homophenyl alanine portion is very expensive. In addition, the expensive S-phenylethyl amine used in the process is completely lost during the processing, further increasing the costs. It would be much more desirable if the S-homophenyl alanine could be coupled with the benzla:ctam portion without racemization and then the desired (S) chirality could be induced at the site alpha to the lactam. These steps are demonstrated in the present invention.

### BRIEF DESCRIPTION OF THE FIGURES

Scheme 1 schematically illustrates the present invention of coupling the S- homophenyl alanine with the benzlactam and the resolution of the two diastereomers which are formed through an epimerization, kinetic resolution step to greatly increase the amount of the desired S,S form.

Scheme 2 illustrates the conversion of (1'S,3S)-3-[(1'-(ethoxycarbonyl)-3'-phenylpropyl)amino]-2,3,4,5-tetrahydro-2-oxo-1*H*-benzazepine to benazepril hydrochloride, an angiotensin-converting enzyme (ACE) inhibitor.

### SUMMARY OF THE INVENTION

One object of the present invention is to demonstrate an efficient process for the preparation of 3-[(S)-1'-(alkoxycarbonyl)-3'-phenylpropyl)amino]-2-oxo-[1] -benzazepine and derivatives thereof. A compound of the formula (III) can be prepared by the reaction of a compound of the formula (I) with a compound of the formula (II), where R₂ is a lower alkyl such as ethyl, proplyl and butyl, under basic conditions in the presence of a phase transfer catalysis in an aprotic polar solvent. The reaction can be further catalyzed by addition of alkali metal halides such as sodium or potassium iodide. 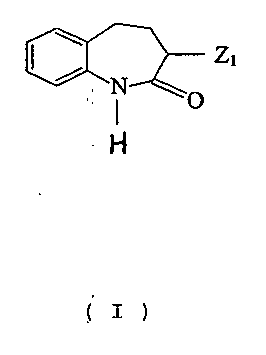 where Z₁= halide such as Br, Cl and I 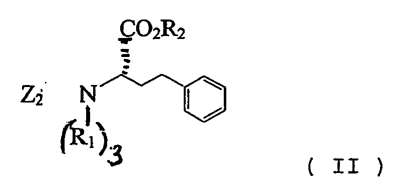 Where R₁= hydrogen, lower alkyl or a combination of hydrogen and alkyl groups
R₂ = a lower alkyl group having 1 to 4 carbon atoms
Z₂ = halide such as Br, Cl and I

In a preferred embodiment, compound of formula (II) is S-homophenylalanine alkyl ester. When S-homophenylalanine alkyl ester is optically pure, the chiral center at the 2-position can be reacted with the compound of formula (I) without racemization to form good yields of compound (III).

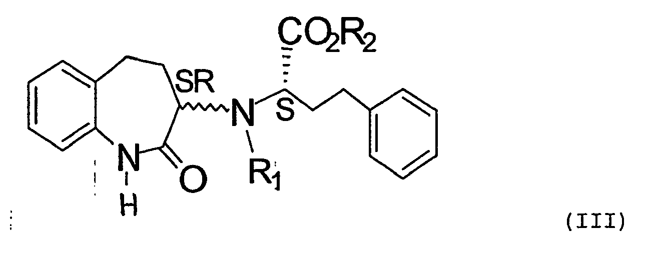

The initial products obtained by the method disclosed therein is therefore a pure mixture of only two diastereomers (hereinafter "S,S/R,S")

By a conventional method, i.e., through simple crystallization, the desired S,S form can be separated. However, a low yield of only approximately 30% is obtained and thus, about 70% of this expensive material would be wasted. It is desired to have a method to recycle the material back to the crystallization. This requires a method to selectively epimerize just the center alpha to the lactam without loss of chirality in the sensitive S-homophenylalanine moiety. Because removal and thus epimerization of the S-homophenylalanine ester proton was shown to be more facile under all conditions explored, we desired a method to accomplish this epimerization in high yield and optical purity. The present invention provides such a method to selectively epimerize the undesired R,S forms to the desired (R,S),S diastereomeric mixture which can then be reused in the crystallization process. Also, surprisingly, the present invention demonstrates a method for the direct conversion of the R,S form to the desired S,S form by kinetic resolution which eliminates the need to recycle the intermediate. This is illustrated schematically, in scheme 1 and discussed below.

An economic process is complicated by the fact that the chiral position on the homophenyl alanine ester is more labile than the benzlactam chiral position under a variety of epimerization conditions. Epimerization occurring on the wrong position would complete destroy the ability to recycle the material. The applicant discovered, however, that an initial conversion of the ester to a carboxylic acid could achieve two desired effects of: (1) deactivating epimerization at the wrong position, i.e., the chiral position on the homophenyl alanine and (2) inducing reaction at the desired position, i.e., the benzlactam chiral position. Thus the R,S diasteriomer of the carboxylic acid compound undergoes an epimerization process under basic or neutral condition resulting in a mixture RS,S diastereomers resulting from the selective epimerization at the lactam position. Furthermore, by careful choice of epimerization and crystallization conditions, a kinetic resolution can be made to occur during which the R,S diasteriomer is converted in high yield directly to the desired S,S diasteriomer. Once the R,S carboxylic acid compound is converted predominantly to the S,S diastereomer, it is converted back to the ester compound without loss of chirality by esterification.

The above described epimerization and kinetic resolution can be achieved under a variety of conditions provided that the desired S,S diastereomer is less soluble in the media. The best results are obtained with the free acid, rather than the acid salts. The epimerization can be made to occur thermally and therefore requires a sufficiently high temperature. The high temperature condition can be achieved by either using a high boiling solvent or by heating the reaction mixture under pressure to increase its boiling temperature. Good results can be achieved in both polar and non-polar solvent systems, as long as the solubility and thermal requirements are met. For example, both xylene and ethylene glycol-water systems are found suitable to conduct the epimerization and chiral induction process. Propionic acid and acetic acid may also be used to conduct the epimerization and chiral induction process. While it is apparent that the process can take place in a variety of solvent systems, aromatic hydrocarbons such as xylene are the preferred solvent. Temperatures between 25°C and 150°C are indicated with higher temperatures necessary in the more non-polar solvents. The results of the epimerization or racemisation studies using various organic solvents are shown in the examples.

The carboxylic acid compound can be converted to the desired ethyl ester compound by re-esterification without loss of chirality. The esterification can be carried out in a number of ways known to those skilled in the art, but the preferred method is by reacting with ethyl bromide and potassium carbonate in a polar solvent such as dimethylacetamide.

The invention will be more specifically understood in terms of the following example, which is exemplary only and do not limit the scope of the invention.

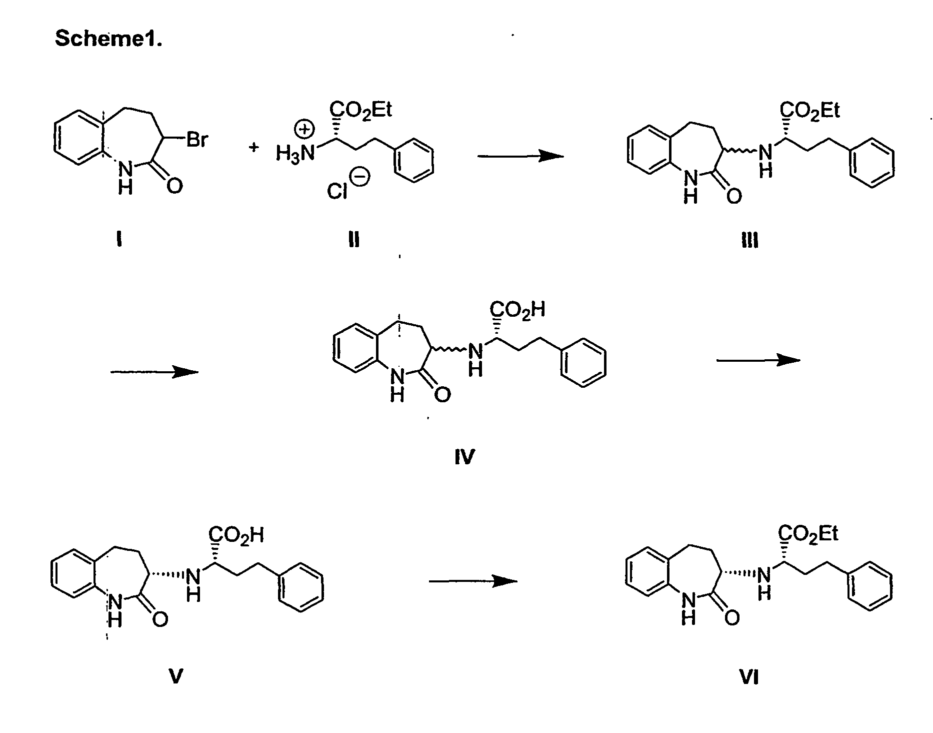

### EXAMPLES

### Example 1

### Step (a)

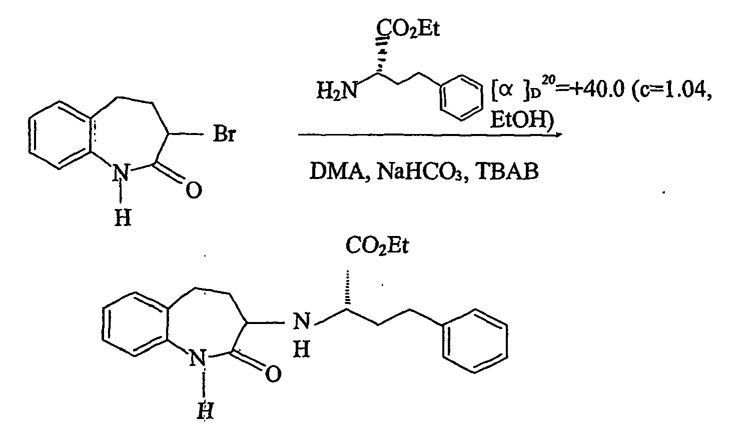

3-Bromo-2,3,4,5-tetrahydro-1H-1-benzazepin-2-one (17.68 g) was prepared by a method analogous to that given in Helvetica Chimica Acta (page 337, vol. 71, 1988). L-homophenylalanine ethyl ester (L-HPAEE, 19.91 g) was prepared, by extracting hydrogen chloride salt of L-homophenylalanine ethyl ester (L-HPAE, HCl, 30g) with ethyl acetate in a solution of sodium carbonate (15 g Na₂CO₃ in 100 ml H₂O).

Sodium bicarbonate (6.84 g), tetra-n-butylammonium bromide (TBAB, 1.191 g), 3-bromo-2,3,4,5-tetrahydro-1H-1-benzazepin-2-one (17.68 g), and N,N-dimethylacetamide (40 ml) were subsequently added to a flask under nitrogen. L-HPAEE was mixed with N,N-dimethylacetamide (30 ml) and the mixture was then added to the flask. The mixture was stirred and heated to 110°C for 6 hours. At this temperature sodium bicarbonate is converted mainly to sodium carbonate.

The mixture was further extracted and analyzed by high pressure liquid chromatography (HPLC) and the results showed that 39.5% of (1'S,3S)-3-[(1'-(ethoxycarbonyl)-3'-phenylpropyl)amino]-2,3,4,5-tetrahydro-2-oxo-1H-benzazepine and 40.31% of (1'S,3R)-3-[(1'-(ethoxycarbonyl)-3'-phenylpropyl)amino]-2,3,4,5-tetrahydro-2-oxo-1H-benzazepine. The desired S,S form of the product could be crystallized out using solvents of ethyl acetate and heptane in the yield from 25% to 33%.

### EXAMPLE 2

The procedure of Example A was repeated, except that sodium iodide (0.96 g) was added to enhance reaction rates in exchanging halogens and to reduce the amount of by-products. The results showed that the mixture contained 40.7% of (1'S, 3S)-3-[(1'-(ethoxycarbonyl)-3'-phenylpropyl)amino]-2,3,4,5-tetrahydro-2-oxo-1H-benzazepine and 41.4% of (1'S, 3R)-3-[(1'-(ethoxycarbonyl)-3'-phenylpropyl)amino]-2,3,4,5-tetrahydro-2-oxo-1H-benzazepine.

### EXAMPLE 3

### (1'S,3R,S)-3-[(1'-carboxyl-3'-phenylpropyl)amino]-2,3,4,5-tetrahydro-2-oxo-1H-benzazepine (IV)

(1'S,3R,S)-3-[(1'-carboxyl-3'-phenylpropyl)amino]-2,3,4,5-tetrahydro-2-oxo-1H-benzapine ethyl ester (III) (2.47kg) was dissolved in 1 liter of methanol. Subsequently, 3N aqueous NaOH (2.4L) was added to reaction mixture and the mixture was stirred for 2 hours at 40'-50°C. The slurry was cooled and 2 N hydrochloric acid (3436 ml) was added to acidify the solution. Methanol was removed by distillation to produce a solid which was filtered, washed with water and dried at reduced pressure to give 1.86 kg of the crude acid (IV).

### EXAMPLE 4

### (1'S,3,S)-3-[(1'-carboxyl-3'-phenylpropyl)amino]-2,3,4,5-tetrahydro-2-oxo-1H-benzazepine (V)

Xylene (30L) was added to 1.86kg of compound IV. The slurry was heated at 150-155°C for 8 hours under approximately 1.5 atm pressure. The reaction mixture was cooled to room temperature. The solid was collected by filtration and dried at reduced pressure to yield 1.67kg of (S,S) diasteriomer (V) as a 98:2 S,S:R,S diasteriomeric mixture as determined by HPLC. The ratio of enantiomers as determined by HPLC is S,S:R,R = 93:7 and the chemical yield was 92%. The compound was characterized as follows: mp 287-290°C; ¹HNMR (DMSO, 400MHz) d1. 63-1.82 (m, 2H), 1.88-2.04(m, 1H), 2.31-2.42(m, 1H), 2.50-2.80(m, 4H), 3.01 (t, J=6.2 Hz, 1H), 3.15 (dd, J=7.8, 11.0 Hz, 1H), 4.02(br, 1H), 6.96(d, J=7.6 Hz, 1H), 7.08-7.16(m, 4H), 7.18-7.31(m, 4H), 9.88 (s, 1H)

### EXAMPLE 5

In a similar manner to example 4, xylene (2.76L) was added to compound IV (83g). The slurry was heated to 138-143°C and maintained at this temperature for 3 hours. After this period, the mixture was cooled to room temperature. The solid was collected by filtration and dried under reduced pressure to give 74.7g of compound V (S,S) as a 97:03 diasteriomeric mixture as determined by HPLC. The ratio of enantiomers as determined by HPLC is SS:RR = 95:05 and the yield is 86%.

### EXAMPLE 6

In a similar manner to example 4, propionic acid (12ml) was added to compound IV (2g). The slurry was stirred at 60 oC for 30 minutes. After this period, the reaction mixture was cooled to 25°C. The solid was collected by filtration, washed with ethyl acetate and dried at reduced pressure to give 1.5g of compound V as a 70:30 diasteriomeric mixture as determined by HPLC. The ratio of enantiomers as determined by HPLC is SS: RR =86:14 and the yield is 75 %.

### EXAMPLE 7

In a similar manner to example 4, acetic acid (6 ml) was added to compound IV (1g). The slurry was stirred at room temperature for 1 hr. The solid was collected by filtration, washed with ethyl acetate and dried at reduced pressure to give 0.7g of compound V as a 99:1 diasteriomeric mixture as determined by HPLC. The ratio of enantiomers as determined by HPLC is SS: RR = 85:15 and the yield is 70 %.

### EXAMPLE 8

In a similar manner to example 4, ethylene glycol (9ml) and H2O (1ml) were added to compound IV (1g). The slurry was heated to 138°C and stirred at this temperature for 3.5 hr. After this period, the reaction mixture was cooled to 25°C. The solid was collected by filtration, washed with ethyl acetate and dried under reduced pressure to give 0.83g of compound V as a 99:01 diasteriomeric mixture as determined by HPLC. The ratio of enantiomers determined by HPLC is SS: RR = 81:19 and the yield is 83 %.

### EXAMPLE 9

(1'S, 3R)-3-[(1'-carboxyl-3'-phenylpropyl)amino]-2,3,4,5-tetrahydro-2-oxo-1H-benzazepine (100mg) and THF (5mL) were mixed and cooled to 0°C. Then the CH30Na (30 mg) was added to the reaction mixture. Then the reaction temperature was kept at 25°C for one hour. The pH was adjusted to 2.5 to 2.0 by 3N HCl solution. The solid was filtered off and dried at reduced pressure to give 93 mg of compound (IV) as a 54:46 S,S:R,S diasteriomeric mixture as determined by HPLC.

### EXAMPLE 10

### (1'S, 3S)-3-[(1-(ethoxycarbonyl)-3'-phenylpropyl)amino]-2,3,45-tetrahydro- 2-oxo-1H-benzazepine (VI)

Compound V (450g), N,N-dimethylacetylamide (2L), bromoethane (115ml), and potassium carbonate (65 g) were added to the reaction flask. The reaction mixture heated to 60-70°C and was stirred for 2 hours at this temperature. The mixture was cooled and 3.5 L of water was added to the mixture at 10°C. The resulting precipitate was collected by filtration, washed with an additional 2L of water and dried at reduced pressure to give 520g of crude solid. This solid was dissolved in a mixture of 0.6L of ethyl acetate and 1.2L of Heptanes at 40-50°C. The solution was cooled to 30°C and the product was isolated by filtration to obtain 390g of VI (S,S) as a >99:1 diasteriomeric mixture as determined by HPLC. The ratio of enantiomers determined by HPLC is SS:RR >99.5 :0.5. and the yield is 80%. The compound is characterized as follows: mp 119-120 oC; [α]²⁰-204° (c=0.99, EtOH), IR(KBr): 3250, 1726, 1671cm-1; IHNMR (CDCl3, 400MHz) d 1.14(t, J=7.2Hz, 3H), 1.91-2.07(m, 3H), 2.43-2.53(m, 2H), 2.59-2.64(m, 2H), 2.68-2.75(m,2H), 2.82-2.92(m, 1H), 3.25-3.35(m, 2H), 4.01-4.11 (m, 2H), 6.95-7.04(m, 1H), 7.10-7.29(m, 8H), 8.64-8.80 (br s, 1H); 13CNMR (CDC13, 50MHz) d 14.1, 28.8, 32.0, 35.0, 37.8, 56.6, 60.0, 60.5, 122.0, 125.8, 125.9, 127.5, 128.2, 129.5, 134.3, 136.5, 141.3, 174.2, 175.2; HRMS, Cal. For C₂₂H₂₆O₃N₂: 366.1945 (M+), found: 366.1950(M+)

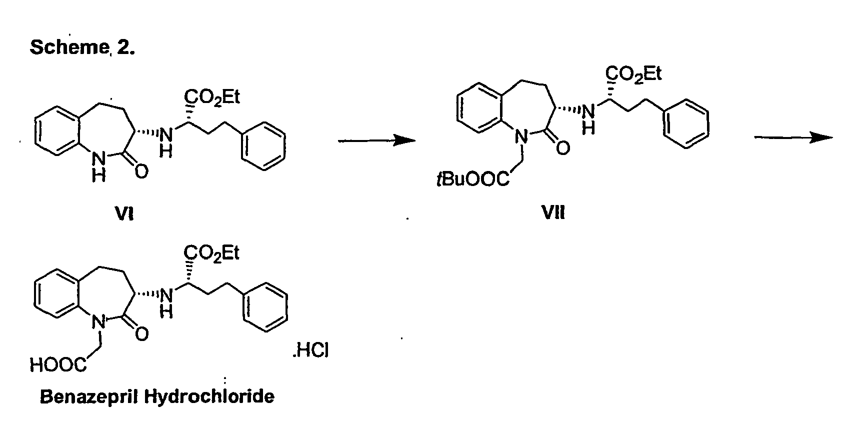

The invention is not limited by the embodiments described above which are presented as examples only but can be modified in various ways within the scope of protection defined by the appended patent claims.

Thus, while there have shown and described and pointed out fundamental novel features of the invention as applied to a preferred embodiment thereof, it will be understood that various omissions and substitutions and changes in the form and details of the devices illustrated, and in their operation, may be made by those skilled in the art without departing from the spirit, of the invention. For example, it is expressly intended that all combinations of those elements and/or method steps which perform substantially the same function in substantially the same way to achieve the same results are within the scope of the invention. Moreover, it should be recognized that the elements and/or method steps shown and/or described in connection with any disclosed form or embodiment of the invention may be incorporated in any other disclosed or described or suggested form or embodiment as a general matter of design choice. It is the intention, therefore, to be limited only as indicated by the scope of the claims appended hereto. All references cited herein are incorporated by reference in their entirety.

## Claims

1. A process for the preparation of a compound of the formula (III) 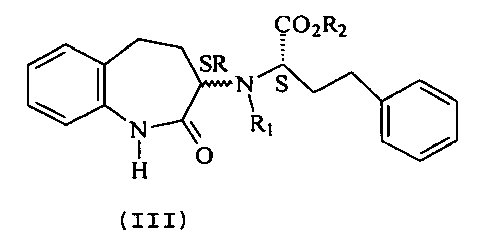 comprising the steps of reacting a compound of the formula (I) 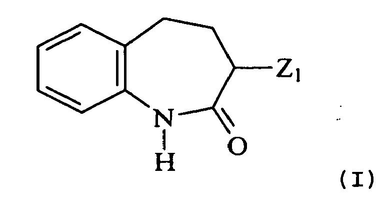 wherein Z1 is a halogen, with a compound of the formula (II) 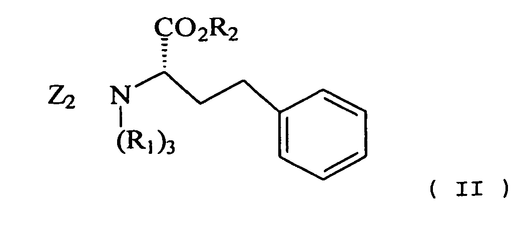 where R₁ is independently selected from hydrogen and lower alkyl;
R₂ is a lower alkyl group having 1 to 4 carbon atoms; and
Z₂ is a halogen;
in an aprotic polar solvent catalyzed by a phase transfer catalyst.

2. The process of claim 1, wherein the phase transfer catalyst is selected from the group consisting of tetra-alkylammonium halides, N-dodecyl-N-methylephedrinium halides, phenyltrimethylammonium halides, phenyltrimethylammonium methosulfate, benzyltrimethylammonium halides, N-benzylcinchoninium halides, benzyldimethyldodecylammonium halides and benzethonium halides.

3. The process of claim 1, wherein the reaction is further catalyzed by an alkali earth metal halide salt.

4. The process of claim 3 wherein the salt is sodium iodide or lithium iodide.

5. The process of the claim 1, wherein the reaction is carried out under basic condition.

6. The process of claim 5 where the basic condition is provided by adding a base selected from the group consisting of sodium bicarbonate, sodium carbonate, potassium bicarbonate, potassium carbonate, and lithium carbonate, and barium carbonate.

7. The process of the claim 1 wherein the reaction temperature is between 60°C and 140°C.

8. A compound of the formula (IV)

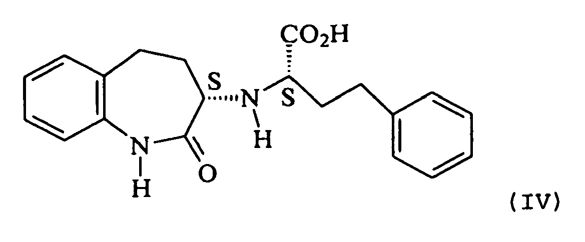

9. A process for making a carboxylic acid of the formula (V) comprising epimerizing IV(R,S) in a solution comprising a polar or non-polar solvent to a compound V where R₁ and R₂ are independently H, benzyl, alkylsilyl or carbamate

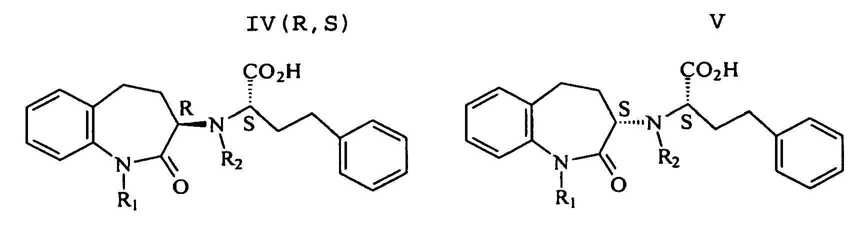

10. The process of claim 9 wherein the epimerization is done by heating the solution.

11. A process of claim 9 in which R₁=H, R₂=H, and epimerization occurs by heating the solution.

12. A process of claim 11 in which the solvent is an alcohol, organic acid or aromatic hydrocarbon.

13. A process of claim 9 in which R₁=H, R₂=H and a base is used to epimerize IV(R,S).

14. The process of claim 13 wherein the base is selected from the group consisting of alkoxide, hydroxide, carbonate and amine.

15. A process of claim 14 in which R₁=H, R₂=H, the base is sodium methoxide, and the solvent is methanol.

16. The process of claim 9, further comprising selectively precipitating the compound of formula V during epimerzation.

17. The process of claim 16 in which the epimerization occurs by heating the solution.

18. The process of claim 17 in which the solvent is an aromatic hydrocarbon or an organic acid.

19. The process of claim 18 wherein the organic acid is acetic acid or propionic acid.

20. The process of claim 18 in which the organic solvent is xylene and the temperature for epimerization is greater than 120°C.

21. The process of claim 9 wherein the carboxylic acid of formula V is converted to an alkyl ester by esterification.

22. The process of claim 21 in which the esterification is effected with an alkyl halide and potassium or sodium carbonate in a organic solvent.

23. A process of claim 22 in which the organic solvent is dimethylacetamide.

24. A process of epimerizing a carboxylic acid compound of formula III 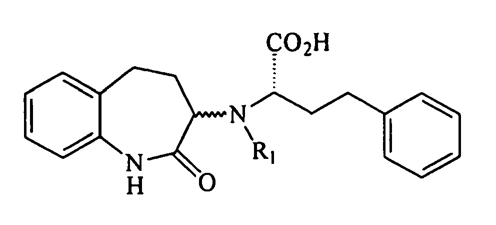 wherein R1 is hydrogen, or a lower alkyl group, to increase the amount of the SS diastereomer of the compound of formula III by heating the carboxylic acid compound of formula III in an organic solvent.

25. The process of claim 24 wherein the SS diastereomer of the compound of formula III is converted by esterification to the ester compound of the formula VI 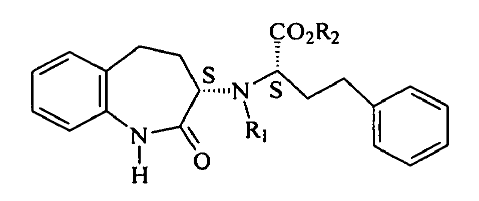 wherein R1 is hydrogen, or a lower alkyl group; and
R2 is a lower alkyl group having 1 to 4 carbon atoms.

26. The process of claim 25 wherein the SS diastereomer of the compound of formula III is converted to the ester compound of formula VI by reacting the compound of formula III with an C₁ to C₄ alkyl halide and potassium carbonate.

27. The process of claim 26 wherein the C₁ to C₄ alkyl halide is ethyl bromide.

28. The process of claim 24 wherein the organic solvent is selected from the group consisting of xylenes, ethylene glycol-water systems, propionic acid and acetic acid.

29. The process of claim 28 wherein the process of epimerizing the carboxylic acid compound of formula III in xylenes further comprises the esterification of the SS diastereomer of formula III by reacting the SS diastereomer of formula III with n,n-dimethylacetylamide, bromoethane and potassium carbonate in said xylenes solution.

30. The process of claim 25 wherein the ester compound of formula VI is further converted to benazepril.

## Patentansprüche

1. Verfahren zur Herstellung einer Verbindung der Formel (III) 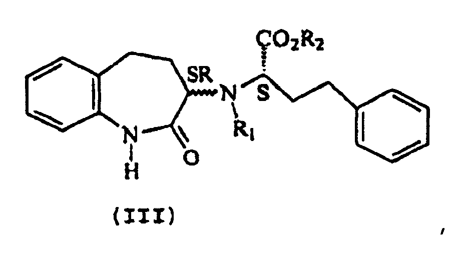 bei dem man eine Verbindung der Formel (I) 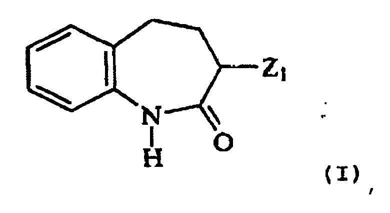 in welcher Z₁ für ein Halogen steht, in einem aprotischen polaren Lösungsmittel katalysiert durch einen Phasentransferkatalysator mit einer Verbindung der Formel (II) 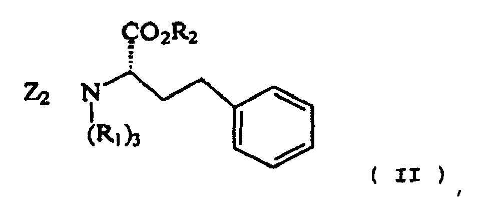 in welcher R₁ unabhängig aus Wasserstoff und Niederalkyl ausgewählt ist;
R₂ für eine Niederalkylgruppe mit 1 bis 4 Kohlenstoffatomen steht und
Z₂ für ein Halogen steht;
umsetzt.

2. Verfahren nach Anspruch 1, wobei der Phasentransferkatalysator ausgewählt ist aus der Gruppe bestehend aus Tetraalkylammoniumhalogeniden, N-Dodecyl-N-methylephedriniumhalogeniden, Phenyltrimethylammoniumhalogeniden, Phenyltrimethylammoniummethosulfat, Benzyltrimethylammoniumhalogeniden, N-Benzylcinchoniumhalogeniden, Benzyldimethyldodecylammoniumhalogeniden und Benzethoniumhalogeniden.

3. Verfahren nach Anspruch 1, wobei die Umsetzung weiter durch ein Erdalkalihalogenidsalz katalysiert wird.

4. Verfahren nach Anspruch 3, wobei es sich bei dem Salz um Natriumiodid oder Lithiumiodid handelt.

5. Verfahren nach Anspruch 1, wobei die Umsetzung unter basischen Bedingungen durchgeführt wird.

6. Verfahren nach Anspruch 5, wobei die basischen Bedingungen durch Zusatz einer Base ausgewählt aus der Gruppe bestehend aus Natriumhydrogencarbonat, Natriumcarbonat, Kaliumhydrogencarbonat, Kaliumcarbonat, Lithiumcarbonat und Bariumcarbonat bereitgestellt werden.

7. Verfahren nach Anspruch 1, wobei die Reaktionstemperatur zwischen 60°C und 140°C liegt.

8. Verbindungen der Formel (IV)

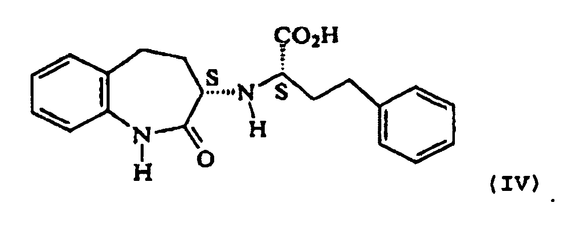

9. Verfahren zur Herstellung einer Carbonsäure der Formel (V), bei dem man IV(R,S) in einer ein polares oder nichtpolares Lösungsmittel enthaltenden Lösung zu einer Verbindung V, in welcher R₁ und R₂ unabhängig für H, Benzyl, Alkylsilyl oder Carbamat stehen, epimerisiert.

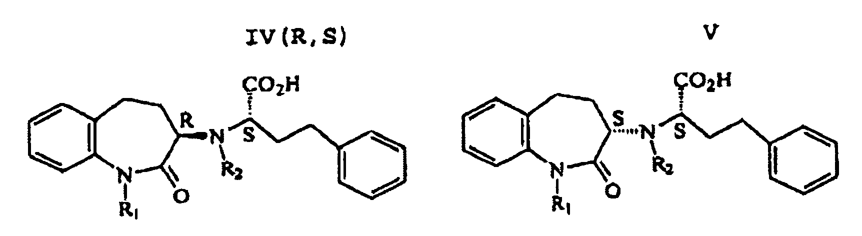

10. Verfahren nach Anspruch 9, wobei die Epimerisierung durch Erhitzen der Lösung erfolgt.

11. Verfahren nach Anspruch 9, wobei R₁ = H, R₂ = H und die Epimerisierung durch Erhitzen der Lösung erfolgt.

12. Verfahren nach Anspruch 11, wobei es sich bei dem Lösungsmittel um einen Alkohol, eine organische Säure oder einen aromatischen Kohlenwasserstoff handelt.

13. Verfahren nach Anspruch 9, wobei R₁ = H, R₂ = H und zur Epimerisierung von IV(R,S) eine Base verwendet wird.

14. Verfahren nach Anspruch 13, wobei die Base ausgewählt ist aus der Gruppe bestehend aus Alkoxiden, Hydroxiden, Carbonaten und Aminen.

15. Verfahren nach Anspruch 14, wobei R₁ = H, R₂ = H, die Base Natriummethanolat und das Lösungsmittel Methanol ist.

16. Verfahren nach Anspruch 9, bei dem man weiterhin während der Epimerisierung die Verbindung der Formel V selektiv ausfällt.

17. Verfahren nach Anspruch 16, bei dem die Epimerisierung durch Erhitzen der Lösung erfolgt.

18. Verfahren nach Anspruch 17, bei dem es sich bei dem Lösungsmittel um einen aromatischen Kohlenwasserstoff oder eine organische Säure handelt.

19. Verfahren nach Anspruch 18, wobei es sich bei der organischen Säure um Essigsäure oder Propionsäure handelt.

20. Verfahren nach Anspruch 18, bei dem es sich bei dem organischen Lösungsmittel um Xylol handelt und die Temperatur für die Epimerisierung über 120°C liegt.

21. Verfahren nach Anspruch 9, wobei die Carbonsäure der Formel V durch Verestern in einen Alkylester umgewandelt wird.

22. Verfahren nach Anspruch 21, wobei die Veresterung mit einem Alkylhalogenid und Kalium- oder Natriumcarbonat in einem organischen Lösungsmittel durchgeführt wird.

23. Verfahren nach Anspruch 22, wobei es sich bei dem organischen Lösungsmittel um Dimethylacetamid handelt.

24. Verfahren zur Epimerisierung einer Carbonsäureverbindung der Formel III 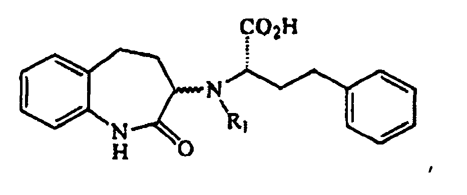 wobei R₁ für Wasserstoff oder eine Niederalkylgruppe steht, zum Erhöhen der Menge an SS-Diastereomer der Verbindung der Formel III durch Erhitzen der Carbonsäureverbindung der Formel III in einem organischen Lösungsmittel.

25. Verfahren nach Anspruch 24, wobei das SS-Diastereomer der Verbindung der Formel III durch Verestern zur Esterverbindung der Formel VI 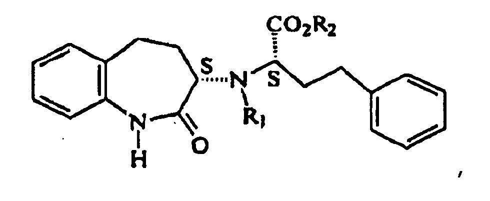 wobei R₁ für Wasserstoff oder eine Niederalkylgruppe steht und
R₂ für eine Niederalkylgruppe mit 1 bis 4 Kohlenstoffatomen steht,
umgewandelt wird.

26. Verfahren nach Anspruch 25, wobei das SS-Diastereomer der Verbindung der Formel III durch Umsetzen der Verbindung der Formel III mit einem C₁- bis C₄-Alkylhalogenid und Kaliumcarbonat in die Esterverbindung der Formel VI umgewandelt wird.

27. Verfahren nach Anspruch 26, wobei es sich bei dem C₁- bis C₄-Alkylhalogenid um Ethylbromid handelt.

28. Verfahren nach Anspruch 24, wobei das organische Lösungsmittel ausgewählt ist aus der Gruppe bestehend aus Xylolen, Ethylenglycol-Wasser-Systemen, Propionsäure und Essigsäure.

29. Verfahren nach Anspruch 28, wobei das Verfahren der Epimerisierung der Carbonsäureverbindung der Formel III in Xylolen weiterhin die Veresterung des SS-Diastereomers der Formel III durch Umsetzen des SS-Diastereomers der Formel III mit N,N-Dimethylacetamid, Bromethan und Kaliumcarbonat in der Xylollösung umfaßt.

30. Verfahren nach Anspruch 25, wobei die Esterverbindung der Formel VI weiter in Benazepril umgewandelt wird.

## Revendications

1. Procédé de préparation d'un composé de formule (III) 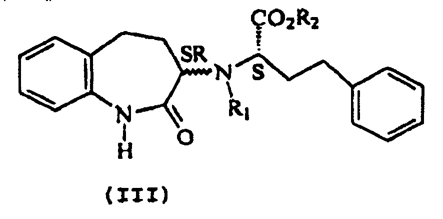 comprenant les étapes consistant à faire réagir un composé de formule (I) 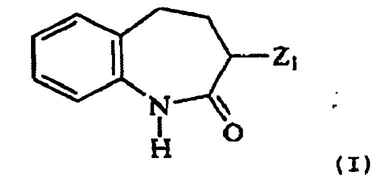 dans laquelle Z₁ représente un atome d'halogène, avec un composé de formule (II) 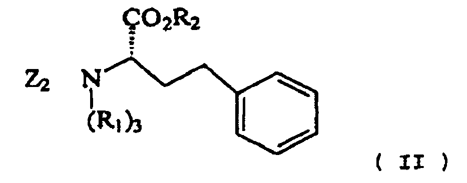 dans laquelle R₁ est choisi indépendamment parmi un atome d'hydrogène et un groupe alkyle inférieur ;
R₂ représente un groupe alkyle inférieur comportant 1 à 4 atomes de carbone ; et
Z₂ représente un atome d'halogène ;
dans un solvant polaire aprotique, avec une catalyse au moyen d'un catalyseur de transfert de phase.

2. Procédé selon la revendication 1, dans lequel le catalyseur de transfert de phase est choisi dans le groupe constitué par les halogénures de tétraalkylammonium, les halogénures de N-dodécyl-N-méthyléphédrinium, les halogénures de phényltriméthylammonium, le méthylsulfate de phényltriméthylammonium, les halogénures de benzyltriméthylammonium, les halogénures de N-benzylcinchoninium, les halogénures de benzyldiméthyldodécylammonium et les halogénures de benzéthonium.

3. Procédé selon la revendication 1, dans lequel la réaction est en outre catalysée par un sel d'halogénure de métal alcalino-terreux.

4. Procédé selon la revendication 3, dans lequel le sel est l'iodure de sodium ou l'iodure de lithium.

5. Procédé selon la revendication 1, dans lequel la réaction est effectuée dans des conditions basiques.

6. Procédé selon la revendication 5, dans lequel on obtient les conditions basiques en ajoutant une base choisie dans le groupe constitué par le bicarbonate de sodium, le carbonate de sodium, le bicarbonate de potassium, le carbonate de potassium, et le carbonate de lithium et le carbonate de baryum.

7. Procédé selon la revendication 1, dans lequel la température réactionnelle est comprise entre 60°C et 140°C.

8. Composé de formule (IV)

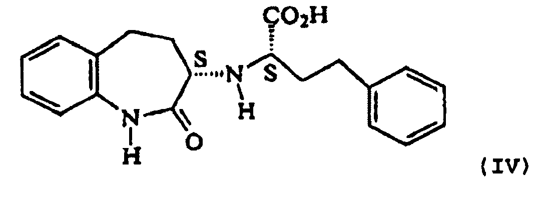

9. Procédé de préparation d'un acide carboxylique de formule (V) comprenant l'épimérisation de IV(R,S) dans une solution comprenant un solvant polaire ou non polaire, en composé V, dans lequel R₁ et R₂ représentent indépendamment H ou des groupes benzyle, alkylsilyle ou carbamate

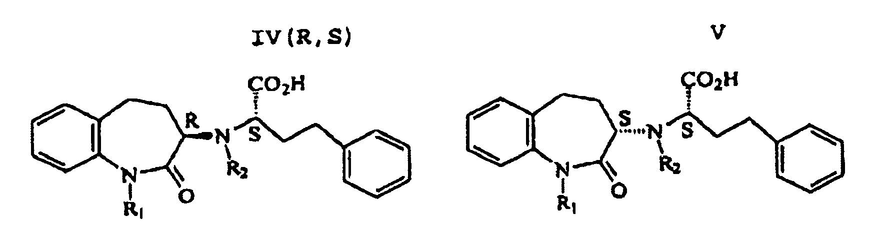

10. Procédé selon la revendication 9 dans lequel l'épimérisation est effectuée par chauffage de la solution.

11. Procédé selon la revendication 9 dans lequel R₁ = H et R₂ = H et l'épimérisation est effectuée par chauffage de la solution.

12. Procédé selon la revendication 11 dans lequel le solvant est un alcool, un acide organique ou un hydrocarbure aromatique.

13. Procédé selon la revendication 9 dans lequel R₁ = H, R₂ = H et on utilise une base pour épimériser IV(R,S).

14. Procédé selon la revendication 13 dans lequel la base est choisie dans le groupe constitué par un alcoolate, un hydroxyde, un carbonate et une amine.

15. Procédé selon la revendication 14 dans lequel R₁ = H, R₂ = H, la base est le méthylate de sodium et le solvant est le méthanol.

16. Procédé selon la revendication 9, comprenant en outre la précipitation sélective du composé de formule V pendant l'épimérisation.

17. Procédé selon la revendication 16 dans lequel l'épimérisation se fait par chauffage de la solution.

18. Procédé selon la revendication 17 dans lequel le solvant est un hydrocarbure aromatique ou un acide organique.

19. Procédé selon la revendication 18 dans lequel l'acide organique est l'acide acétique ou l'acide propionique.

20. Procédé selon la revendication 18 dans lequel le solvant organique est le xylène et la température de l'épimérisation est supérieure à 120°C.

21. Procédé selon la revendication 9 dans lequel l'acide carboxylique de formule V est transformé en ester alkylique par estérificaton.

22. Procédé selon la revendication 21 dans lequel l'estérification est effectuée avec un halogénure d'alkyle et du carbonate de potassium ou de sodium dans un solvant organique.

23. Procédé selon la revendication 22 dans lequel le solvant organique est le diméthylacétamide.

24. Procédé d'épimérisation d'un composé acide carboxylique de formule III 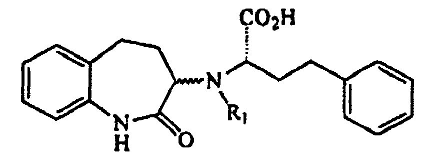 dans laquelle R₁ représente un atome d'hydrogène ou un groupe alkyle inférieur, pour augmenter la quantité du diastéréoisomère SS du composé de formule III, consistant à chauffer le composé acide carboxylique de formule III dans un solvant organique.

25. Procédé selon la revendication 24 dans lequel le diastéréoisomère SS du composé de formule III est transformé, par estérification, en composé ester de formule VI 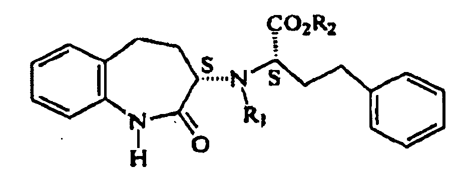 dans laquelle R₁ représente un atome d'hydrogène ou un groupe alkyle inférieur ; et
R₂ représente un groupe alkyle inférieur comportant 1 à 4 atomes de carbone.

26. Procédé selon la revendication 25 dans lequel le diastéréoisomère SS du composé de formule III est transformé en composé ester de formule VI par réaction du composé de formule III avec un halogénure d'alkyle en C₁ à C₄ et du carbonate de potassium.

27. Procédé selon la revendication 26 dans lequel l'halogénure d'alkyle en C₁ à C₄ est le bromure d'éthyle.

28. Procédé selon la revendication 24 dans lequel le solvant organique est choisi dans le groupe constitué par les xylènes, les systèmes éthylèneglycol/eau, l'acide propionique et l'acide acétique.

29. Procédé selon la revendication 28 dans lequel le procédé d'épimérisation du composé acide carboxylique de formule III dans les xylènes comprend en outre l'estérification du diastéréoisomère SS du composé de formule III par réaction du diastéréoisomère SS du composé de formule III avec du N,N-diméthylacétylamide, du bromoéthane et du carbonate de potassium dans ladite solution dans les xylènes.

30. Procédé selon la revendication 25 dans lequel le composé ester de formule VI est encore transformé en benazépril.
